# EUROPEAN PATENT APPLICATION

(11) **EP 0 080 822 A1**
(43) Date of publication of application: **08.06.1983**
(21) Application number: 82306092.6
(22) Date of filing: 16.11.1982
(51) Int. Cl.: C07C 103/52, A61K 37/02

(54) **Anti-hypertensive prolinol-based peptides**

(30) Priority: 27.11.1981 GB 8135949; 15.01.1982 GB 8201100; 08.03.1982 GB 8206752
(71) Applicant: BEECHAM GROUP PLC, Brentford, Middlesex TW8 9BD (GB)
(72) Inventor: Evans, John Morris, Roydon Essex (GB); Humphries, Alfred John, Newport Essex (GB); Markwell, Roger Edward, Great Dunmow Essex (GB)
(74) Representative: Stott, Michael John (GB)

(57) **Abstract**

Compounds of formula (I): 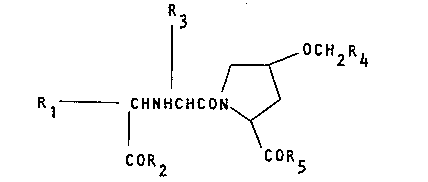
or a pharmaceutically acceptable salt thereof, wherein R₁ is C₁₋₅ alkyl optionally substituted by NHR₆, (wherein R₆ is hydrogen or C₁₋₅ alkylcarbonyl) or by phenyl or naphthyl optionally substituted by halogen, C₁₋₅ alkyl or C₁₋₅ alkoxy or by dihydrobenzofuran-2-yl, optionally substituted in the benzo moiety by C₁₋₅ alkyl, C₁₋₅ alkoxy, hydrogen or trifluoromethyl;
R₂ and R₅ are the same or different and each is hydroxy, C₁₋₅ alkoxy, C₂₋₆ alkylcarbonyl or amino optionally substituted by C₁₋₅ alkyl;
R₃ is C₁₋₅ alkyl optionally substituted by the group -NHR₇, wherein R₇ is hydrogen, C₁₋₅ alkyl or C₂₋₆ alkylcar- bonvl; and
R₄ is phenyl optionally substituted by halogen, C₁₋₅ alkoxy, trifluoromethyl or C₁₋₅ alkyl having antihypertensive activity, a process for their preparation and their use.

## Description

This invention relates to novel compounds having pharmacological activity, to pharmaceutical compositions containing them, and to a process for their preparation.

Captopril is a known compound having anti-hypertensive activity and the formula (A):

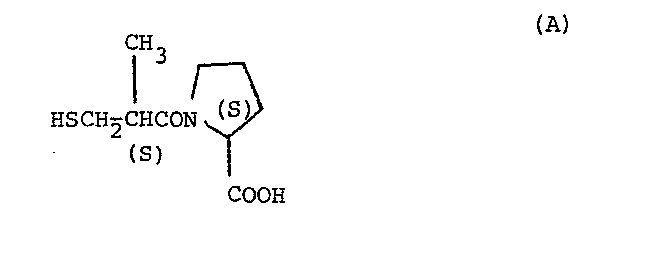

European Patent Publication No. 12 401 describes a class of compounds which also have anti-hypertensive activity and which differ from captopril by the replacement of the HSCH₂-moiety by a group of formula (B): 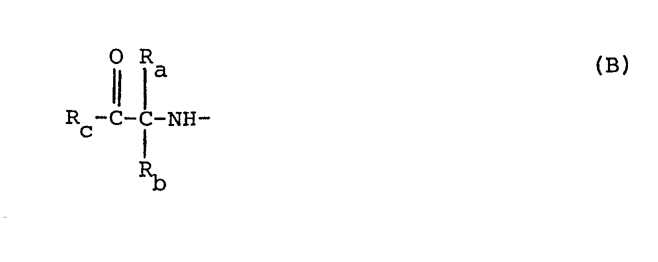 wherein
Rₐ is hydrogen, alkyl, substituted lower alkyl, substituted or unsubstituted aryl, substituted or unsubstituted arloweralkyl, arloweralkenyl, heteroarloweralkyl or heteroarloweralkenyl, or arloweralkyl or heteroarloweralkyl substituted on the alkyl position, and
R_{b} is hydrogen or lower alkyl, and
R_{c} is hydroxy or alkenoxy or alkoxy, aryloxy, or amino, each of which may-be optionally substituted.

A representative compound disclosed in the European Patent Publication has formula (C): 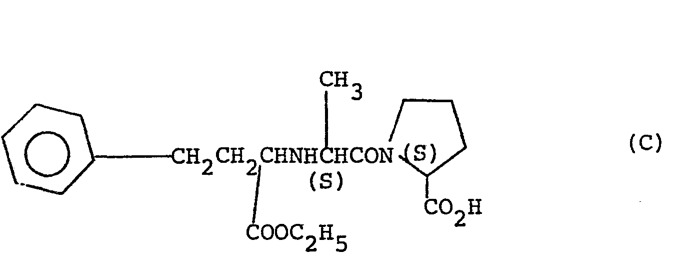 and is referred to as N-(1(S)-ethoxycarbonyl-3-phenylpropyl)-L-alanyl-L-proline.

It has now been found that certain novel armethyleneoxy- substituted compounds also have anti-hypertensive activity.

Accordingly, the present invention provides a compound of formula (I): 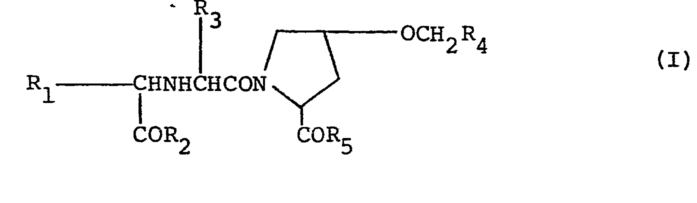
or a pharmaceutically acceptable salt thereof, wherein R₁ is C₁₋₅ alkyl optionally substituted by NHR₆, (wherein R₆ is hydrogen or C₁₋₅ alkylcarbonyl) or by phenyl or naphthyl optionally substituted by halogen, C₁₋₅ alkyl or C₁₋₅ alkoxy or by dihydrobenzofuran-2-yl, optionally substituted in the benzo moiety by C₁₋₅ alkyl, C₁₋₅ alkoxy, halogen or trifluoromethyl;
R₂ and R₅ are the same or different and each is hydroxy, C₁₋₅ alkox^{y}, C₂₋₆ alkylcarbonyl or amino optionally substituted by C₁₋₅ alkyl;
R₃ is C₁₋₅ alkyl optionally substituted by the group -NHR₇, wherein R₇ is hydrogen, C₁₋₅ alkyl or C₂₋₆ alkylcarbonyl; and
R₄ is phenyl optionally substituted by halogen, C₁₋₅ alkoxy, trifluoromethyl or C₁₋₅ alkyl.

Favourably, R₁ is C₁₋₅ alkyl, such as methyl, ethyl, n- propyl, and iso-propyl or C₁₋₅ alkyl such as ethyl, substituted by phenyl or methyl,or propyl, substituted by dihydrobenzofuran-2-yI. Preferably R₁ is ethyl, n-propyl, phenethyl or n-propyl substituted by dihydrobenzofuran-2-yl.

Preferred examples of R₂ and R₅ include hydroxy, methoxy, ethoxy, and n- and iso-propoxy. Often R₅ is hydroxy and R₂ is hydroxy or ethoxy.

Preferred examples of R₃ are unsubstituted C₁₋₅ alkyl groups, such as methyl, ethyl, n- and iso-propyl and the amino-substituted alkyl groups, -(CH2)nNH2, wherein n is from 1 to 4 for example 1, 2 or 4.

A preferred example for R₄ is unsubstituted phenyl.

The pharmaceutically acceptable salts of the compounds of formula (I) include those with bases, such as alkali metal and alkaline earth metal salts; for example sodium and potassium salts and ammonium salts; and those with acids, such as hydrochloride, hydrobromide, sulphate, phosphate, maleate and like salts.

There is a group of compounds within formula (I) wherein R₁ is Cₗ-₅ alkyl optionally substituted by -N_{H}R₆ wherein R₆ is hydrogen or C₂-₆ alkylcarbonyl.

From the aforesaid it will be appreciated that a group of compounds of formula (I) of interest is that of formula (II): 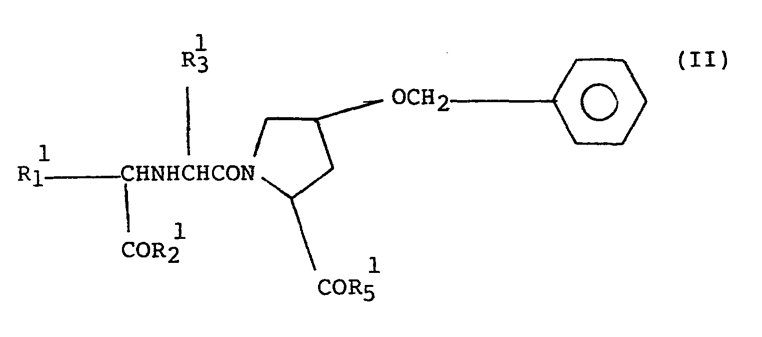 wherein:
R₁¹ is Cₗ-₅ alkyl optionally substituted by phenyl or dihydrobenzofuran-2-yl; 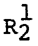 is Cₗ-₅ alkoxy or hydroxy; R₃¹is C₁₋₅ alkyl; and R₅^{l}is hydroxy.

A preferred sub-group of compounds within formula (II) is of formula (III): 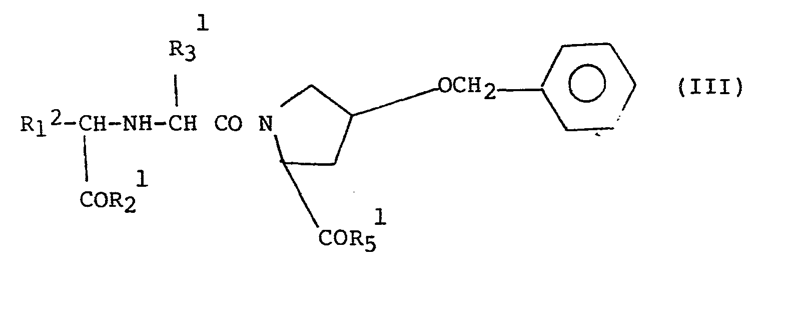 wherein R₁² is a Cₗ-₅ alkyl group and the remaining variables are as defined in formula (II).

Favourable values for R₁² are as described for relevant R₁ under formula (I). Preferred values for R₁² are ethyl, iso-propyl and sec-butyl, most preferably ethyl and n- propyl.

Another preferred sub-group of compounds within formula (II) is of formula (IV): 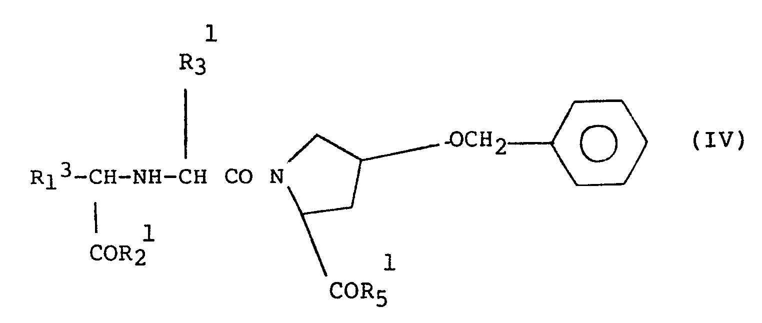 wherein R₁³ is C₁₋₃ alkyl substituted by phenyl and the remaining variables are as defined in formula (II).

R₁³ is preferably phenethyl.

Another sub-group within formula (II) is of formula (V): 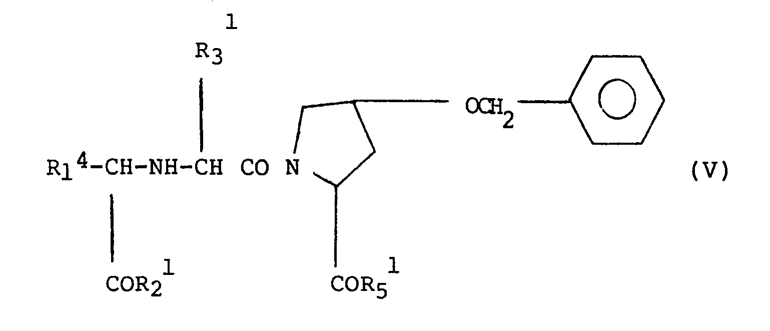 wherein R₁⁴ is Cₗ₋₃ alkyl substituted by dihydrobenzofuran-2-yl and the remaining variables are as defined in formula (II).

Preferred values for R₁⁴ are dihydrobenzofuran-2-yl methyl and dihydrobenzofuran-2-yl propyl.

The compounds of formula (I) are inhibitors of angiotensin converting enzyme, and thus have antihypertensive activity. They may accordingly be used in the therapy of hypertension in mammals, such as humans.

Accordingly, the present invention also provides a pharmaceutical composition, which comprises a compound of formula (I) or, in particular of formula (II), and a pharmaceutically acceptable carrier.

The compositions of this invention are most suitably adapted for oral administration although adaption for other modes of administration for example by injection, are also possible.

In order to obtain consistency of administration it is preferred that the compositions of this invention are in the form of a unit-dose. Suitable unit-dose forms include tablets, capsules, ampoules and powders in sachets. Such unit-dose forms aptly contain from 1 to 100 mg of the compound of the invention and more usually from 2 to 75 mg, for example 5 to 50 mg.

Such compositions may be administered from 1 to 6 times a day, more usually from 2 to 4 times a day, in a regimen such that the daily dose is from 5 to 200 mg for a 70 kg human adult and preferably from 10 to 100 mg.

The compositions of this invention may be formulated in conventional manner, for example in a manner similar to that used for known anti-hypertensive agents such as hydrallazine.

In addition such compositions may contain further active agents such as other anti-hypertensive agents especially a-blocking agents, and diuretics.

The invention also provides a method of treatment of hypertension in mammals including humans which method comprises the administration of a compound of formula (I) or a pharmceutically acceptable salt thereof.

The invention also provides a process for the preparation of a compound of formula (I) which process comprises the reduction of a compound of formula (VI): 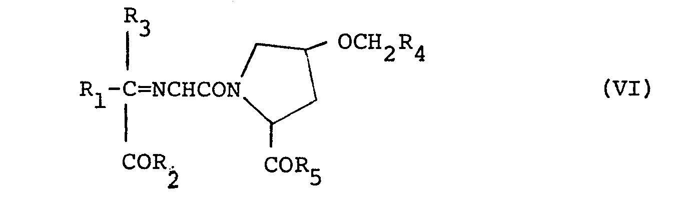 wherein R₁ to R₅ are as defined in formula (I). The reduction is carried out in any suitable manner known generally for such reductions. For example sodium cyanoborohydride may be used in a suitable dry solvent, such as ethanol.

Alternatively the reaction may be carried out by hydrogenation over one of the conventional catalysts, such as palladium or carbon or platinum or rhodium in a suitable dry solvent for example ethanol.

The compounds of formula (III) which are novel intermediates and represent part of the invention, may in turn be prepared by reacting a compound of fornula (VII)_{:} 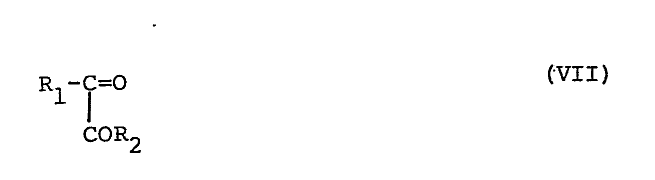 with a compound of formula (VIII) 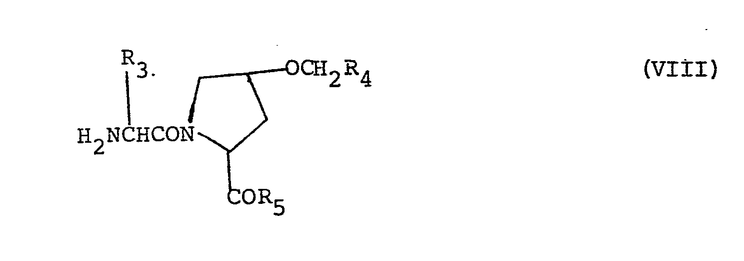 wherein _{R1} to R₅ are as defined in formula (I).

The coupling reaction between the compounds of formulae (VII) and (VIII)may be carried out by mixing together the reactants in a dry solvent.

The two-step conversion of the compounds of formulae (VII) and(VIII)into the desired compound of formula (I) or (II) may preferably be carried out in one operation by producing the imine of formula (VI) in situ. In such case , a means for removing the water formed as a by-product of imine formation should be present, such as molecular sieves. The reduction of the imine and the removel of the water will drive the reaction forward to give the desired product of formula (I): the actual amount of imine formed at any time being very small.

The compounds of formulae (VII) and (VI_{I}I) are either known compounds of may be prepared by processes analogous to those used for known structurally similar compounds.

A modification of the literature method provided by R. Adams and R. E. Rindfusz in J. Am. Chem. Soc. 41, 648 (1919) and H. Normant, Ann. Chim. 17, 335 (1942) is suitable for the preparation of those compounds of formula (_{VII}), wherein the dihydrobenzofuran moiety is bonded to the rest of the structure at the 2-position, and Y is bromo and m is 0 and n is 1. This synthesis is shown below schemetically:

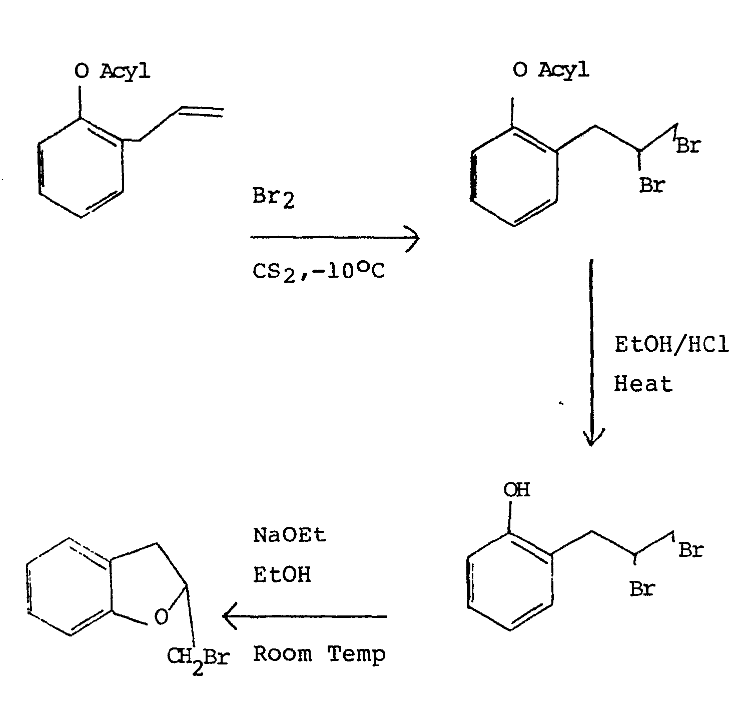

After the preparation of a compound of formula (I) as herein described certain variable groups in the compound may then be optionally converted to other groups. By way of example, a compound of formula (I), wherein R₂ and R₅ are both hydroxy, may be esterified in conventional manner to give the corresponding compound of formulla (I), wherein R₂ and R₅ are both alkoxy.

The salts of the compounds of formula (I) and (II) may be prepared in conventional manner, for example by reacting the compound of formulae (I) and (II) with acid or base as appropriate.

The compounds of formula (I) and (II) have asymmetric centres and thus are capable of existing in a number of stereoisomeric forms. This invention extends to each of these stereoisomeric forms and to mixtures thereof (including racemates). The different stereoisomeric forms may be separated one from the other by conventional techniques or any given isomer may be obtained by a stereospecific synthesis.

The asymmetric centres indicated by '^{*}' in the part structures: 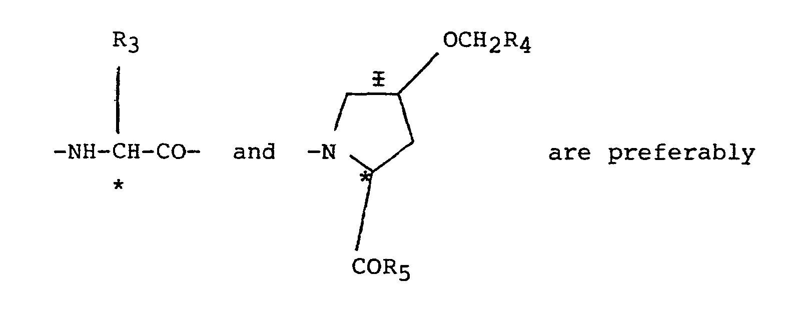 in the S configuration. The asymmetric centre indicated by 'I' in the pyrrolidino ring above may have and- or β- configuration. However, the α-configuration is preferred.

The asymmetric centre indicated by '^{*}' in the amino acid part structure: 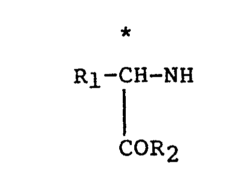 may be in the R and/or S configuration, preferably in the S configuration or in both configurations together as in a racemic mixture.

In addition, when R₁ is alkyl substituted by optionally substituted dihydrobenzofuran-2-yl, then there is a fifth asymmetric centre indicated by "^{*}" in the part structure.

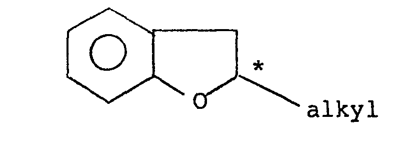

The structure may be in the R and/or S configuration, preferably in both configurations together as in a racemic mixture.

The following Examples illustrate the invention.

### Example 1

### Preparation of N-(1-Carboxypropyl)-(S)-alanyl-4α-benzyloxy-(S)-proline

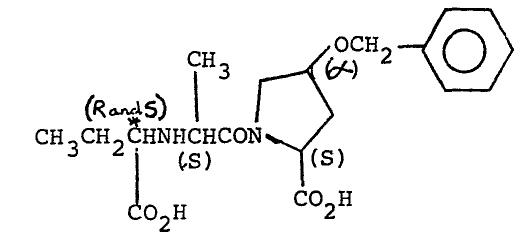

A solution of (S)-alanyl-4-benzyloxy-(S)-proline (0.75 g) & a-ketobutyric acid (1.03 g) in water (30 ml) was adjusted to pH 7 with sodium hydroxide solution. To this stirred solution under nitrogen was added sodium cyanoborohydride (0.38 g) and the stirring continued for 48 hours at room temperature. The reaction mixture was added to Dowex 50-W ion exchange resin (20 g). Elution with water followed by pyridine (2%) in water, and collection of the last aqueous fraction and combination with the basic fractions and evaporation gave a gum (750 mg). The gum was purified using a chromatotron (2 mm silicia gel _{P}F ₂₅₄ plate; solvent flow rate 6 ml/min); elution with methanol-chloroform (3:1) mixture gave the title compound as a white solid (530 mg).

NMR (D₂O) δ 0.88 (3H, br.t);
1.47 (3H, d);
1.82 (3H, m);
2.35 (lH, m);
3.65 (3H, m);
3.80-4.85. (6H, m), overlapping
4.45 (2H, s);
7.34 (5H, br. s).

Mass Spectrum [M⁺⁻H] at ^{m}/_{z} 377 (negative ion F.A.B.)

### Example 2

### Preparation of N-(1-carbethoxy-3-phenylpropyl)-(S)-alanyl-4α-benzyloxy-(S)-proline:

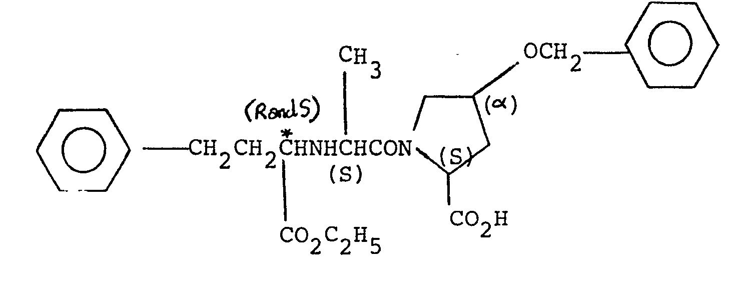

(S)-Alanyl-4-benzyloxy-(S)-proline hydrochloride (0.70 g) was dissolved in dry ethanol (20 ml) containing triethylamine (0.22 g). 4^{g} molecular sieves (2.50 g) and ethyl4-phenyl-2-ketobutyrate (0. 88g) were added to the solution and the resulting mixture stirred at room temperature for 0.5 hr. Sodium cyanoborohydride (0. 19 g) was added in portions during 1.5 hrs. The reaction was stirred overnight before a further 0.44 g of the keto ester was added. Stirring at room temperature for 6 days was followed by filtration and addition with stirring of Dowex 50-W ion exchange resin (30 g) to the filtrate. After 0.5 hr the suspension of resin was applied to a chromatography column. Elution with ethanol water, and pyridine (2%) in water gave a mixture (600 mgm) isolated from the basic fraction. Purification using a chromatotron (2 mm silicia gel PF₂₅₄ plate; solvent flow rate 6 ml/min) eluted with methanol-hlomform (1:3) mixture gave the title compound as a colourless glass (380 mgm).

IR (film) 1725,163₀ cm⁻¹
NMR (CDCl₃) 0.95 - 1.45 (irreg. m, 6H)
1.70 - 4.80 (series of broad multiplets, 14H) overlapping
4.16 (irreg. q, 2H) and 4.40 (S,2H)
7.22 (m, 10H)
Mass spectrum (EI) M⁺ -H₂0 at ^{m}/_{Z} 464.2305 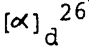
= -44.0° (methanol C = 1)

### Example 3

### Preparation of N-(l-carboxy-3-phenylpropyl)-(S)-alanyl-4 -benzyloxy-(S)-proline

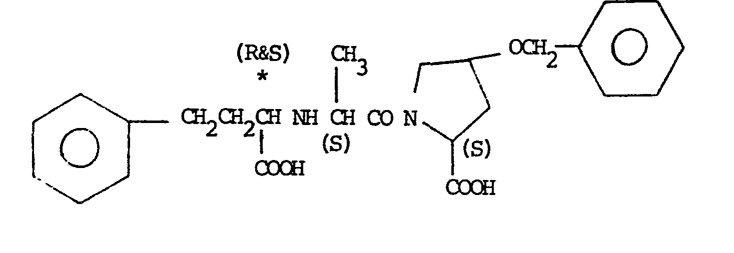

The compound of Example 2 (365 mg), 10% aqueous sodium hydroxide solution (0.6 ml), and ethanol (6 ml) were stirred for 16 hours at room temperature. The organic phase was dried over anhydrous sodium sulphate, filtered and evaporated to give a gum, which on trituration with diethyl ether gave the title diacid as a white solid (230 mg)

Mass spectrum. M⁺⁻H₂0 at 436.2001

[α]_{d}²⁶ = -27.5⁰ (methanol, c = 1)

### Example 4

### Preparation of N-[2-(2,3-dihydro-2-benzofuranyl)-l-(ethoxycarbonyl) -ethyl)-(S)-alanyl -4α-benzyloxy-(S)-nroline

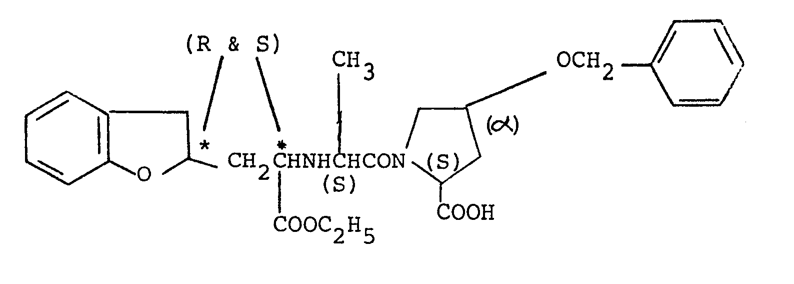

(S)-Alanyl-4α-benzyloxy-(S)-proline hydrochloride (0.5 g) was dissolved in dry ethanol (20 ml) and triethylamine (0.16 g) added. To this solution was added powdered 4Å molecular sieves (3.0 g) and ethyl 2,3-dihydro-3-(2-benzofuranyl)-2- katopropionate (0.73 g) and the resulting mixture stirred under nitrogen, at room temperaturefor 0.5 hr. Sodium cyanoborohydride was added in portions over 3 hours. After stirring for 3 days the reaction was filtered

and Dowex 50-W ion exchange resin (25 g) added to the filtrate. This was applied to a chromatography column after stirring for 1 hour and eluted with ethanol, water and 2% pyridine in water in succession. The basic fraction yielded a mixture (300 mg) which was purified using a chromatotron (₂ mm silica g_{el PF254'} solvent flow rate 6ml/min).

Elution with methanol-chloroform (1:10) gave the title compound as an off white solid (90 m^{g}) after trituration with pentane.

NMR (CDCl₃) 6 0.95 - 1.50 (irreg. m, 6H)
1.55 - 5.15 (series of br m, 15H) overlapping
4.15 (irreg. m, 2H) and 4.44 (s, 2H)
6.55 - 7.50 (irreg. m, 4H)
7.27 (s, 5H)

Mass spectrum M⁺⁻H₂0 at M/_{z}492.2273

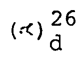

= -51.9° (methanol, c=1)

### Example 5

### Preparation of N-[4-(2,3-dihydro-2-benzofuranyl)-l-(ethoxycar-α bonyl)-butyl]-(S)-alanyl-4αbenzyloxy-(S)-proline

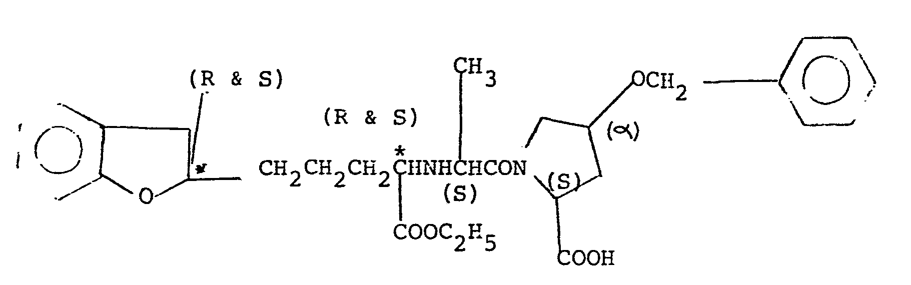

A solution of ethyl 2,3-dihydro-5-(2-benzofuranyl) 2- ketopentan- oate (6.0 g) in dry ethanol (10 ml) was added to a stirred suspension of (S)-alanyl-4α -benzyloxy=(S)-proline hydrochloride (2.0 g), triethylamine (0.5 ml) and powdered activated 4^{g} molecular sieves (22 g) in dry ethanol (40 ml) under nitrogen at room temperature. After 1.5 hr sodium cyanoborohydride (0.43 g) was added portionwise over 30 hr, and at the end of 48 hr the mixture was filtered. Dowex 50-W ion exchange resin (60 g) was added to the filtrate and stirred for 1.5 hr. Transfer to a column was followed by successive elution with ethanol, water and 2% pyridine in water solution.

Evaporation of the relevant pyridine-water fractions gave an oil which was purified by chromatography (silica, 5% methanol-chloroform) to give the title compound (1.₃ mg) as a solid.

M⁺-H₂O at M/_{z} 520.2571

### Example 6

### N-[4-(2,3-dihydro-2-benzoturanyl)-1-carboxybutyl-(S)-alanyl-4 -benzyloxy-(S)-proline

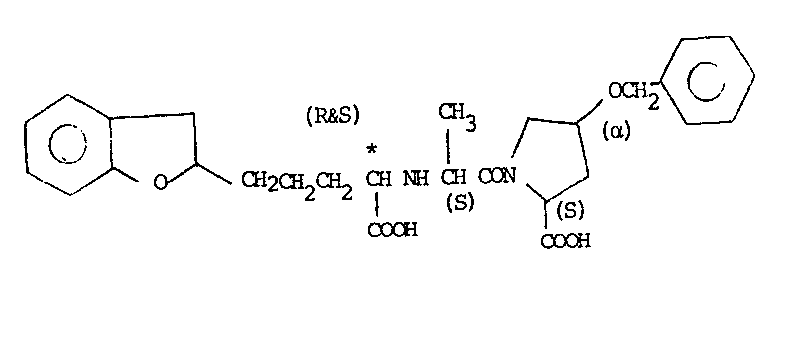

The compound of example 5 (0.45g) and sodium hydroxide pellets (0.067g) were stirred in ethanol (7 ml) at room temperature for 2 days. The solution was neutralised with 20% citric acid and extracted with chloroform. The chloroform was dried with anhydrous Na₂S0₄, filtered, and evaporated to give the title compound (0.25g) as a chromatographically homogeneous solid.

Mass spectrum. M⁺⁻H₂0 at ^{m}/_{Z} 492.2300.

### Example 7

### Preparation of N(l-carbethoxy-5-amino-n-pentyl)-(S)-alanyl-4α-benzyloxy-(S)-proline

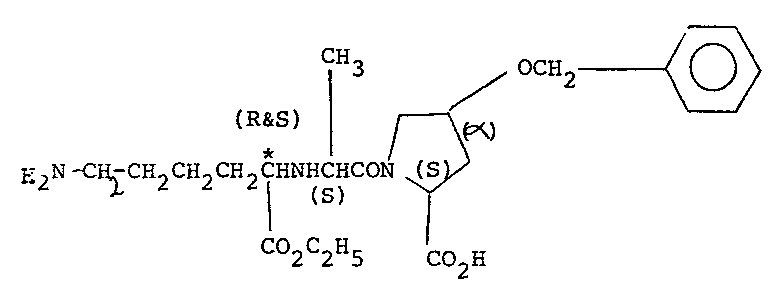

This compound is prepared in substantially the same manner as the preparation of the compound of Example 1, except that the terminal amino group is optionally protected prior to reduction with sodium cyanoborohydride and then de-protected.

### Example 8

Preparation of N-(l-carbethoxy-2-methylpropyl)-2-(S)-alanyl-4 -benzyloxy-(S)-proline.

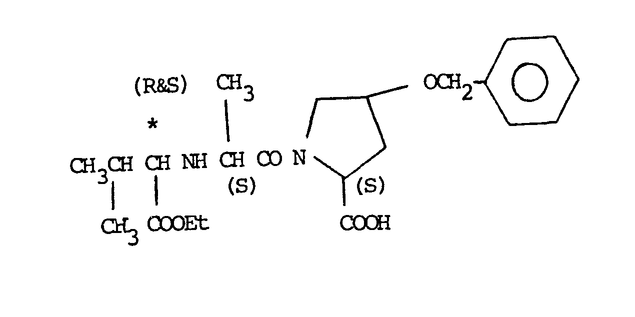

To a solution of (S)-alanyl-4-benzyloxy-(S)-proline hydrochloride (0.50g), and triethylamine (0.16g) in ethanol (30ml) was added powdered 4A molecular sieves (3.0g) and ethyl 3-methyl-2- ketobutyrate (1.5g). To this stirred suspension was added sodium cyanborohydride (O.llg) during 3 hrs. Stirring was continued for 4 days before filtration and addition of Dowex 50-W ion exchange resin. (30g). The resin was successively washed with ethanol, water and pyridine (2%) in water. Evaporation of the aqueous pyridine washing yielded a crude gum which was purified on the chromatotron (2mm) silica gel PF₂₅₄; elution rate 6ml/min). Elution with methanol-chloroform (ascending methanol concentration to 50%) gave the required title compound (0.26 mg).

Mass spectrum. M+ at ^{m}/^{z} 420.2262.

[α]_{d}²⁶ = -48.6⁰ (methanol), c = 1)

### Example 9

### Preparation of N-(l-carboxy-2-methylpropyl)-(S)-alanyl-4 -benzyloxy-(S)-proline

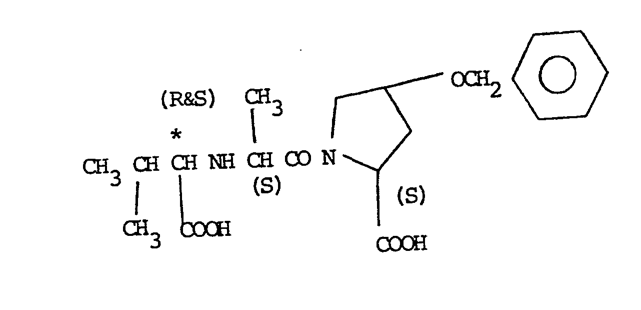

The compound of example 8 (80 mg) was treated with aqueous sodium hydroxide solution (2 equivalents during 24 hours. Acidification with 20% citric acid solution to pH 3.5, extraction with chloroform and addition of Dowex solution exchange resin (5.0 g) to the aqueous phase was followed by elution with water. Elution with pyridine (2%) in water and collection and evaporation of those fractions containing the most polar component gave the title diacid (40 mg).

Mass spectrum. M⁺⁻H₂0 at ^{m}/_{z} 374.1838

[α]_{d}²⁶ = -46.1⁰ (methanol, c = 1)

### Example 10

### N-(l-carbethoxy-3-methyl-butyl)-(S)-alanyl-4 -benzyl- oxy-(S)-proline

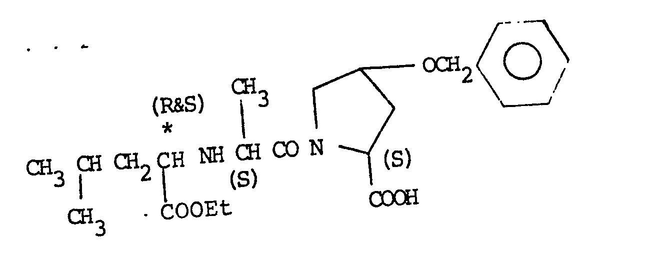

To a solution of (S)-alanyl-4-benzyloxy-(S)-proline hydrochloride (2.0 g), and triethylamine (0.93g) in ethanol (40ml) was added powdered 40A molecular pieces (8.0g), followed by ethyl 2-keto-4-methyl-pentanoate (1.46g) and sodium cyanoborohydride (0.58g) in portions. The reaction mixture was stirred for 4 days, and then filtered and evaporated. The residue was taken up in chloroform and washed with sodium before drying over magnesium sulphate and evaporation. The crude material thus obtained was purified using a chromatotron (2mm silica gel PF₂₅₄; solvent flow rate 6ml/min). Elution with methanol-chloroform (1:3) gave the title compound as a chromatographically homogenous solid (0.33 g).

Mass spectrum showed M⁺-H₂O at ^{m}/z 416 (EI) and _{MH}^{+ m}/z 435 (Ammonia CI)

[α]_{d}²⁶= -71.8° (methanol, c = 1)

### Example 11

### N-(l-carboxy-3-methylbutyl)-(S)-alanyl-4 -benzyloxy-(S)-proline

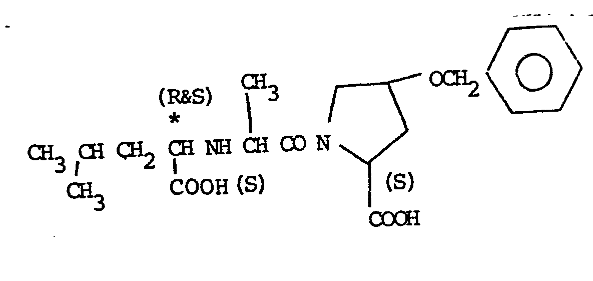

The compound of example 10 (298mg) and sodium hydroxide pellets (55mg) were stirred in ethanol (8ml) at room temperature for 16 hours. The solution neutralised with 20% aqueous citric acid and extracted with chloroform. The organic phase was dried with MgS0₄, filtered, and evaporated and the resulting gum triturated with ether to give the title compound as a white powder (170 mg).

Mass spectrum. M⁺⁻H₂0 at ^{m}/z 388.2011.

[α]_{d}²⁶ = -21.2° (methanol, c = 1)

### Example 12

Preparation of N-(l-carbethoxypropyl)-(S)-alanyl-4 - benzyloxy-(S)-proline.

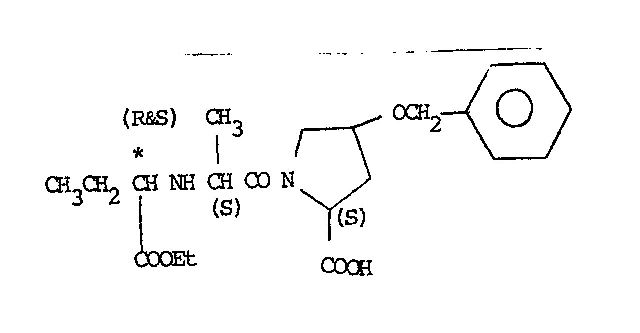

To a solution of (S)-alanyl-4-benzyloxy-(S)-hydrochloride (1.0 g) and triethylamine (0.32 ml) in ethanol (20ml) was added powdered 4A molecular sieves (5 g) and ethyl 2-ketobutyrate (1.7 g). Sodium cyanoborohydride (0.2) was added to the stirred suspension during 3 hrs, and stirring was then continued for 5 days before filtration.

_{D}owex 50-W ion exchange resin (20 g) was added to the reaction mixture and the resin eluted with ethanol, water and then by pyridine (2%) in water. These basic fractions containing product were combined and evaporated and purified with a chromatotron (2 mm silica gel PF₂₅₄: solvent flow rate 6 ml/mm ). Elution with methanol-chloroform (2:3) mixture gave the title compound (0.33g) as a solid.

Mass spectrum. M⁺H₂0 at ^{m}/_{z} 388.2006.

[α]_{d}²⁶ = -70.2 (methanol, c = 1)

### Example 13

### Preparation of N-(1-Carboxybutyl)-(S)-alanyl-4-benzyloxy-(S)-proline

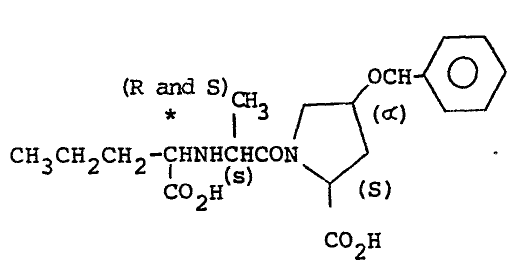

The title compound was prepared in an analogous manner to the compound of Example 1 using -ketopent- anoic acid instead of -ketobutyric acid.

Mass spectrum. [M⁺⁻H₂0] at ^{m}/_{Z} 374.1855

[α]_{d}²⁶ = -37.99⁰ (in methanol, c = 1)

### PHARMACOLOGICAL DATA

### 1. In vitro test for inhibition of angiotensin converting enzyme

The compound of Example 1, N-(l-carboxypropyl)-(S)-alanyl-4-α(-benzyloxy-(S)-proline, was found to cause a 50% inhibitior. (IC₅₀) of rat lung angiotensin converting enzyme preparation at a concentration of 3.3 x 10⁻⁹ M (mean of 3 experiments).

### 2. In vivo test for inhibition of angiotensin converting enzyme.

The compounds of examples 1,2,4 & 5 were each tested in anaesthetised rats for their ability to reduce the pressor responses to angiotensin I, but not those to angiotensin II. The dose of angiotensin I was 300 n.g/kg (i.v) and the dose of angiotensin II was 100 ng/kg (i.v).

The results given are the mean of those obtained from the given number of rats.

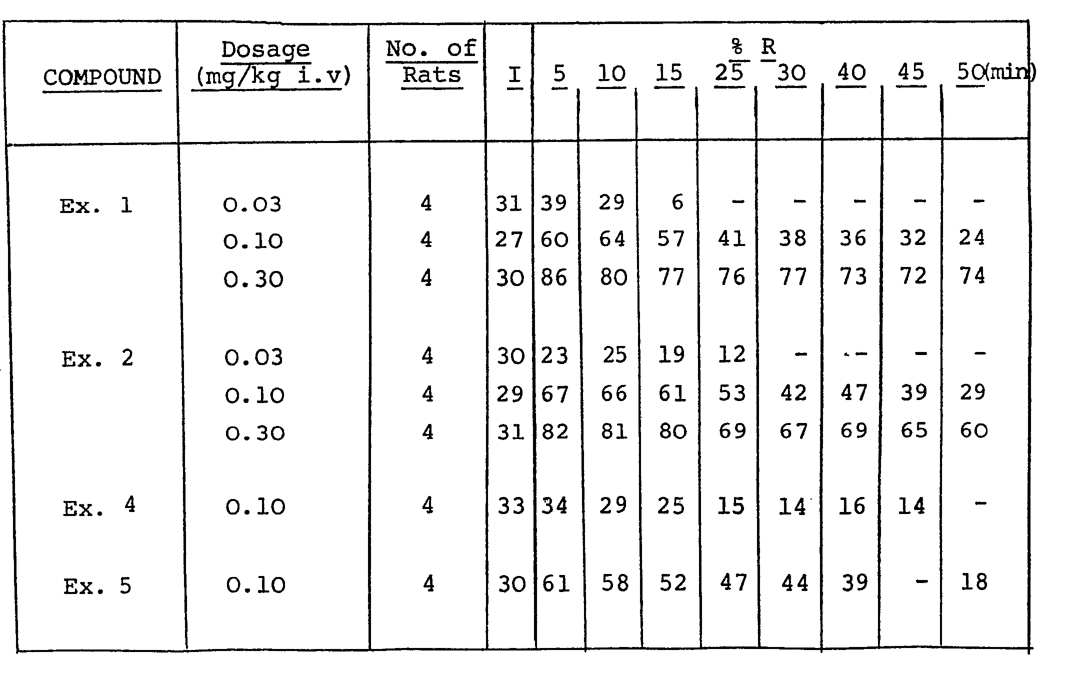

'I' is the increase in diastolic blood pressure (mm Hg) to angiotensin I (control reponse).

'%R' is the percentage reduction in control angiotensin I response after the intervals (min) from dosage.

Examples1,2,4 & 5 slightly augmented the pressor responses to angiotensin II.

From the above results, it is concluded that the compounds of Examples 1,2,4 &'S reduce the pressor responses to angiotensin I, but not those to angiotensin II and thus inhibit angiotensin converting enzyme.

### 3. Antihypertensive Activity

Systolic blood pressures were recorded by a modification of the tail cuff method described by I.M. Claxton, M.G. Palfreyman, R.H. Poyser and R. L. Whiting, European Journal of Pharmacology, 37, 179 (1976).

A W&W BP recorder, model 8005 was used to display pulses. Prior to all measurements rats were placed in a heated environment (33.5 + 0.5°C) before transfer to a restraining cage. Each determination of blood pressure was the mean of at least 6 readings. Spontaneously hypertensive rats (ages 12-18 weeks) with systolic blood pressures 170 mmH:g were considered hypertensive.

The compound of Example 2, N-(l-carbethoxy-3-phenyl propyl)-(S)-alanyl-4-a-benzyloxy-(S)-proline, was administered p.o. to rats at a dose of 10 mg/kg, and the compound of Example 5 N[4[2,3,-dihydro-2-benzofuranyl)-1-(ethoxycarbonyl)-butyl]-(S)-alanyl-4a-benxyloxy-(S)-proline, was administered p.o. to rats at a dose of 30 mg/kg. The initial blood pressure and heart rats were determined and recorded together with the % changes occuring at intervals thereafter:

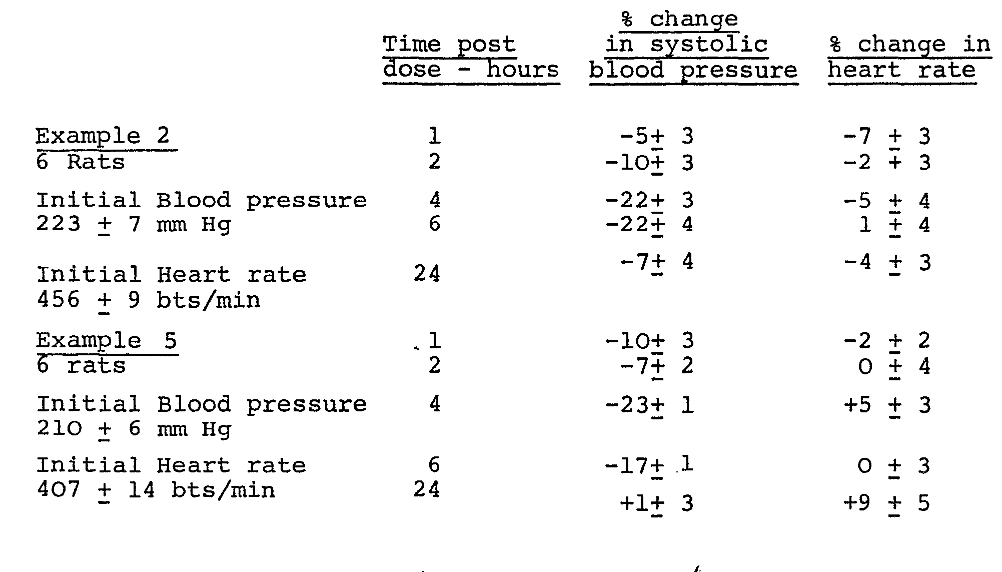

### Toxicity

No toxic effects were observed in the above tests.

## Claims

1. A compound of the tormula (I): 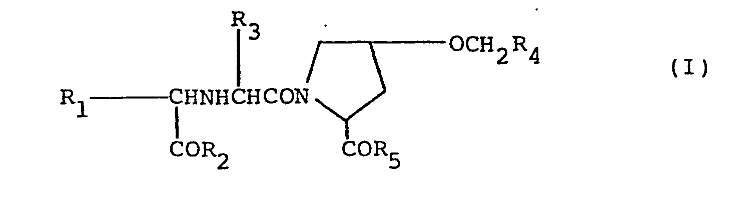
or a pharmaceutically acceptable salt thereot, wherein R₁ is C₁₋₅ alkyl optionally substituted by NHR₆, (wherein R₆ is hydrogen or C₁₋₅ alkylcarbonyl) or by phenyl or naphthyl optionally substituted by halogen, C₁₋₅ alkyl or C₁₋₅ alkoxy or by dihydrobenzofuran-2-yl optionally substituted in the benzo moiety by Cₗ-₅ alkyl, C₁₋₅ alkoxy, halogen or tritluoromethyl;
R₂ and R₅ are the same or ditterent and each is hydroxy, C₁₋₅ alkoxy, C₂₋₆ alkylcarbonyl or amino optionally substituted by C₁₋₅ alkyl;
R₃ is C₁₋₅ alkyl optionally substituted by the group -NHR₇, wherein R₇ is hydrogen, C₁₋₅ alkyl or C₂₋₆ alkylcarbonyl; and
R₄ is phenyl optionally substituted by halogen, Cₗ-₅ alkoxy, trifluoromethyl or C₁₋₅ alkyl.

2. A compound according to claim 1 wherein R₁ is C₁₋₅ alkyl optionally substituted by NHR₆.

3. A compound according to claim 1 of tormula (II): 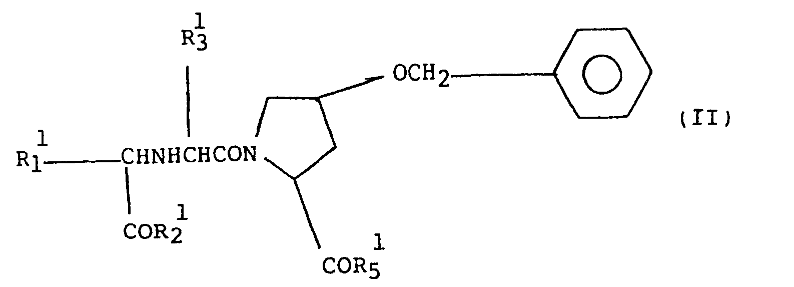 wherein:
H₁¹ is C₁₋₅ alkyl optionally substituted by phenyl or aihydrobenzoturan-2-yl; R₂¹ is C₁₋₅ alkoxy or hydroxy; R₃¹ is Cₗ-₅ alkyl; and R₅¹ is hydroxy.

4. A compound according to claim 2 or 3 of formula 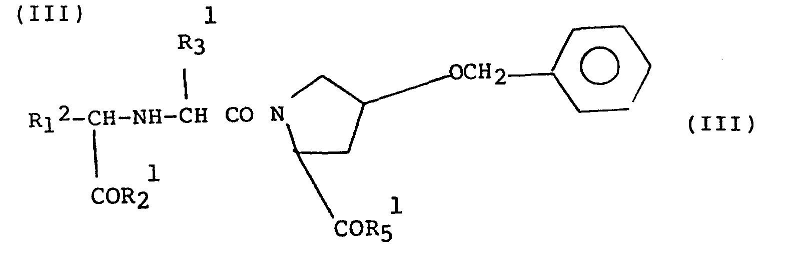 wherein R₁² is a Cₗ-₅ alkyl group and the remaining variables are as defined in claim 3.

5. N-(l-Carboxypropyl)-(S)-alanyl-4 -benzyloxy-(S)-proline; N-(1-Carboxybutyl)-(S)-alanyl-4 -benzyloxy-(S)-proline or N-(l-Carbethoxy-2-methylpropyl)-2-(S)-alanyl-4 -benzyloxy-(S)-proline.

6. A compound according to claim 3 of formula (IV): 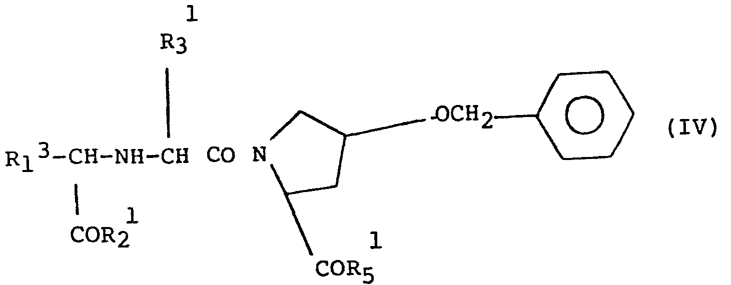 wherein R₁³ is Cₗ-₃ alkyl substituted by phenyl and the remaining variables are as defined in claim 3.

7. N-(1-Carbethoxy-3-phenylpropyl)-(S)-alanyl-4 - benzyloxy-(S)-proline or N-[2-(2,3-dlhydro-z-benzofuranyl)-1-(ethoxycabonyl)-ethyl)-(S)-alanyl-4 - benzyloxy-(S)-proline.

8. A compound according to claim 3 of formula (_{V}): 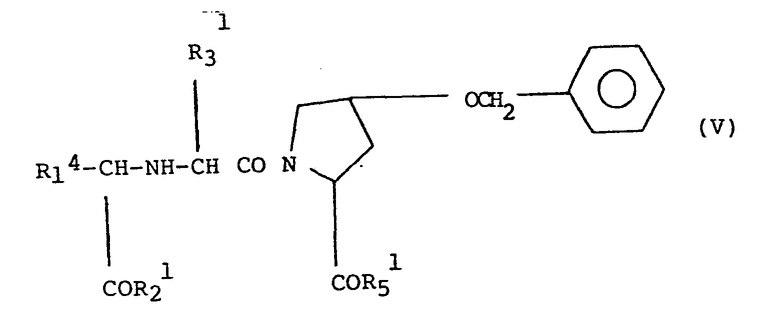 wherein R₁⁴ is C₁₋₃ alkyl substituted by dihydrobenzo- turan-2-yl and the remaining variables are as defined in claim 3.

9. N-[-(2,3-Dihydro-2-benzoturanyi)-1-(ethoxycarbonyl)-ethylJ-(S)-alanyl-4 -benzyloxy-(S)-proline; N-[2-(2,3-dihydro-2-benzofuranyl)-1-(ethoxycarbonyl)-ethyl)-(S)-alanyl-4 -benzyioxy-(S)-proline; N-[4-(2,3-dihydro-2-benzofuranyl)-l-(ethoxycarbonyl)-butyl]-(S)-alanyl-4 -benzyioxy-(S)-proline or N-[4-(2,3-dihydro-2-benzofuranyl)-1-carboxybutyl-(S)-alanyl-4-benzyloxy-(S)-proime.

10. A process tor the preparation ot a compound according to any one of the claims 1 to 8 characterised by the reduction of a compound of formula (vl): 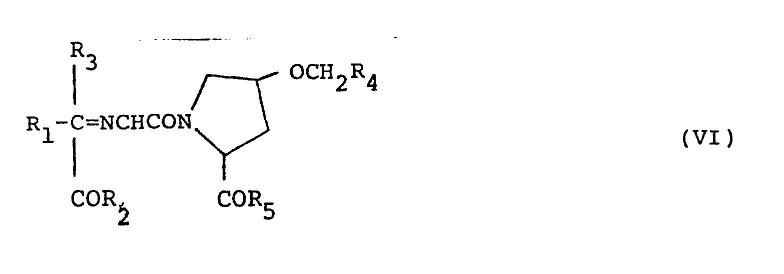 wherein R₁ to R₅ are as detined in claim 1.

11. A pharmaceutical composition which comprises a compound according to any one of the claims 1 to 9 or a pharmaceutically acceptable salt thereof and a pharmaceutically acceptable acceptable carrier.

12. A compound according to any one ot claims 1 to 9 for use in treating hypertension in mammals.
